(19) **Europäisches Patentamt / European Patent Office / Office européen des brevets**

(11) **EP 4 433 353 B1**

(12) **FASCICULE DE BREVET EUROPEEN**

(45) Date de publication et mention de la délivrance du brevet:
**08.07.2026 Bulletin 2026/28**

(21) Numéro de dépôt: **22817255.7**

(22) Date de dépôt: **14.11.2022**

(51) Classification Internationale des Brevets (IPC):
*B62M 3/08* (2006.01)   *B62J 45/421* (2020.01)
*B62J 45/411* (2020.01)   *B62J 45/413* (2020.01)
*B62J 45/414* (2020.01)   *B62J 43/20* (2020.01)
*B62J 43/30* (2020.01)   *A61B 5/22* (2006.01)
*A61B 5/11* (2006.01)   *G01L 5/22* (2006.01)
*G01L 3/24* (2006.01)   *A61B 5/00* (2006.01)

(52) Classification Coopérative des Brevets (CPC):
**B62M 3/08; A61B 5/1121; A61B 5/221; A61B 5/6895; B62J 43/20; B62J 43/30; B62J 45/411; B62J 45/413; B62J 45/414; B62J 45/421; G01L 3/24; G01L 5/225;** A61B 5/6829

(86) Numéro de dépôt international:
**PCT/EP2022/081815**

(87) Numéro de publication internationale:
**WO 2023/084086 (19.05.2023 Gazette 2023/20)**

(54) **PÉDALE CONNECTÉE DE VÉLO**

VERBUNDENES FAHRRADPEDAL

CONNECTED BICYCLE PEDAL

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **15.11.2021 FR 2112056**

(43) Date de publication de la demande:
**25.09.2024 Bulletin 2024/39**

(73) Titulaire: **Sportoptim**
**33100 Bordeaux (FR)**

(72) Inventeur: **DOUSSET, Simon**
**33100 BORDEAUX (FR)**

(74) Mandataire: **Aquinov**
**35, Rue Tourat**
**33000 Bordeaux (FR)**

(56) Documents cités:
EP-A1- 2 072 387   EP-A1- 3 733 493
WO-A1-2012/038677   WO-A1-2021/105951
DE-A1- 10 221 519   DE-U1- 202018 002 389
FR-A1- 2 950 428   FR-A1- 3 078 158

**Description**

Domaine technique.

L'invention se rapporte au domaine technique des pédales connectées.

L'invention a pour objet une pédale connectée de vélo.

Etat de la technique.

Il existe depuis plusieurs années différents types de pédales connectées que l'on peut monter sur un vélo, à la place des pédales classiques. Ce type de pédale est notamment appelé pédale automatique de par leur mise en fonctionnement mécanique par un utilisateur portant des chaussures avec une semelle rigide comportant une cale adaptée pour coopérer avec la pédale. Ainsi ces pédales automatiques peuvent être mises en mouvement par un simple mouvement des pieds de l'utilisateur.

Un inconvénient de ce type de pédale est qu'il existe plusieurs types de pédales adaptées à chaque discipline et donc à chaque type de vélo, par exemple des pédales adaptées pour les vélos sur route et des pédales adaptées pour les vélos tous terrains, voire pour chaque marque de vélo. Un utilisateur possédant ces deux types de vélos a donc besoin de posséder une pluralité de types de pédales, les coûts d'achat sont donc multipliés par le nombre de paires de pédales.

FR 3 078 158 A1 divulgue une pédale connectée selon le préambule de la revendication 1.

L'invention se place donc dans ce contexte et cherche à résoudre l'ensemble des inconvénients précités. Ainsi, l'invention cherche à proposer une pédale connectée comportant un axe standard et intervertible d'un type de vélo à un autre et dont la conception est simplifiée et peu énergivore.

Présentation de l'invention.

L'invention a pour objet une pédale connectée de vélo comportant un corps comportant une plateforme formant une pédale, un axe creux sur lequel la plateforme est montée mobile en rotation, l'axe comportant une portion support de section carrée ou rectangulaire, la pédale étant remarquable en ce qu'elle comporte deux jauges de déformation montées sur l'axe creux, les jauges étant montées sur des faces voisines de ladite portion support, lesdites faces voisines étant montées à 90° l'une de l'autre, lesdites faces voisines étant appelées première face et deuxième faces, et au moins un gyromètre monté sur une troisième face de ladite portion support de l'axe creux, cette troisième face étant distincte des première et deuxième faces supportant les jauges de déformation ; et la pédale étant remarquable en ce qu'elle comporte au moins une unité de calcul agencée pour déterminer une valeur relative à une force exercée par un cycliste sur la pédale à partir des mesures acquises par les jauges de déformation et le gyromètre.

La plateforme est distincte de l'axe creux. En effet, la plateforme peut être montée sur l'axe creux de manière amovible. La plateforme montée sur une pédale gauche peut être sensiblement la même que la plateforme montée sur une pédale droite..

L'axe creux peut être utilisé sur un vélo de type route mais également sur un vélo de type tout terrain, seulement la plateforme la plus adaptée au type de vélo est changée.

La portion support de l'axe creux comporte une section dans laquelle deux faces voisines forment un angle de 90° l'une par rapport à l'autre. Ainsi, la portion support comporte une section carrée ou rectangulaire. La portion support présente ainsi une première face et une deuxième face, perpendiculaires entre elles. La portion support présente une troisième face et une quatrième face, perpendiculaires entre elles et distinctes de la première et de la deuxième face. La troisième face est opposée et parallèle à l'une des première et deuxième faces. Dans un mode de réalisation, la troisième face est opposée et parallèle à la première face et la quatrième face est opposée et parallèle à la deuxième face.

L'utilisation d'un axe creux standard et interchangeable quelle que soit le type de vélo sur lequel il est monté peut permettre de réduire les couts de fabrication et de production. Les coûts d'achat sont également diminués du fait de l'interchangeabilité des axes creux selon le type de vélo. Ce type d'axe peut également permettre de changer uniquement la plateforme en fonction du type de vélo et non toute la pédale, ce qui peut permettre de réduire les coûts puisque la partie électronique de la pédale est absente de la plateforme.

La pédale comporte deux jauges de déformation montées sur la portion support de l'axe creux. Les jauges de déformation peuvent également être appelées jauges de contrainte ou jauges de pression. Lors de la déformation de l'axe creux, les jauges de déformation peuvent se déformer de la même manière. En se déformant, la résistance électrique des jauges de déformation peut varier, cette variation traduisant la déformation de l'axe creux. La lecture de cette résistance par l'unité de calcul, directement ou indirectement, peut ainsi permettre de déterminer ladite valeur relative à une force exercée par un cycliste sur la pédale.

Les deux jauges de déformation sont montées à 90° l'une de l'autre sur des faces voisines, à savoir sur la première et sur la deuxième face. Les jauges de déformation montées à 90° l'une de l'autre peuvent permettre de mesurer la force normale à la rotation de la pédale en se dispensant de la composante radiale. Les jauges de déformation peuvent permettre de mesurer la force exercée par le cycliste sur la pédale pour entrainer sa rotation.

La pédale comporte un gyromètre monté sur la troisième face de la portion support de l'axe creux. Le gyromètre peut permettre de mesurer la vitesse angulaire donnée par le cycliste à la pédale.

La pédale comporte une unité de calcul agencée pour déterminer à partir des données acquises par les jauges de déformation, une force que le cycliste exerce sur la pédale du vélo. L'unité de calcul peut être montée sur l'une des troisième ou quatrième face de la portion support.

Une pédale connectée ayant ce type de configuration peut permettre de maximiser le nombre de pièces communes aux pédales droites et gauches. Il est ainsi possible de diminuer les coûts de fabrication, de production et de vente.

Avantageusement, la pédale connectée comporte quatre jauges de déformation montées sur l'axe creux, deux jauges de déformation étant montées sur la première face en faisant un angle entre elles et deux autres jauges de déformation étant montées sur la deuxième face en faisant un angle entre elles.

La pédale peut comporter quatre jauges de déformation montées sur la portion support de l'axe creux. Les jauges de déformation peuvent être montées deux à deux sur une même face de la portion support. Les quatre jauges de déformation peuvent être montées deux à deux à 90° l'une de l'autre sur des faces voisines. Par exemple, deux jauges peuvent être montées à 90° l'une de l'autre sur la première face, formant un premier capteur de déformation et deux jauges peuvent être montées à 90° l'une de l'autre sur la deuxième face formant un deuxième capteur de déformation.

Avantageusement, la pédale connectée comporte une première carte de circuit imprimé et une deuxième carte de circuit imprimé, montées sur l'axe creux, la première carte de circuit imprimé étant montée sur la troisième face de la portion support et la deuxième carte de circuit imprimé étant montée sur une quatrième face de la portion support, la troisième et la quatrième face étant voisines. De préférence, la première et la deuxième carte de circuit imprimé sont reliées l'une à l'autre par un connecteur flexible.

La première carte de circuit imprimé peut être équipée d'un ou plusieurs composants électroniques, notamment un ou plusieurs amplificateurs opérationnels, aptes à amplifier des signaux émis par lesdites jauges de déformation, notamment par les quatre jauges de déformation. La deuxième carte de circuit imprimé peut être équipée d'au moins ladite unité de calcul, laquelle est agencée pour convertir lesdits signaux amplifiés issus de la première carte de circuit imprimé, ces signaux étant des signaux analogiques, en des signaux numériques et pour déterminer ladite valeur relative à une force exercé par un cycliste sur la pédale à partir desdits signaux numériques.

De préférence, l'unité de calcul est agencée pour déterminer, à partir desdits signaux numériques, en plus de ladite valeur relative à une force exercée par un cycliste sur la pédale, au moins une autre valeur relative au pédalage du cycliste, et notamment une valeur relative à une cadence de pédalage du cycliste, une valeur relative à l'efficacité du cycliste et/ou une valeur relative à la fluidité du coup de pédale.

La première carte de circuit imprimé et la deuxième carte de circuit imprimé étant reliées entre elles par un connecteur flexible peuvent former une pièce unique dont une partie est montée sur la troisième face de la portion support et une autre partie est montée sur la quatrième face de la portion support.

Avantageusement, le gyromètre est monté sur la première carte de circuit imprimé et la pédale connectée comporte un accéléromètre monté sur la deuxième carte de circuit imprimé. Le cas échéant, l'accéléromètre et le gyromètre peuvent faire partie d'un même dispositif, par exemple une centrale inertielle, montée sur la deuxième carte de circuit imprimé.

La pédale peut comporter une unique carte de circuit imprimé montée sur l'une des troisième ou quatrième faces. Le gyromètre et l'accéléromètre pouvant être montés sur ladite unique carte de circuit imprimé.

Dans un autre mode de réalisation, le gyromètre peut être monté sur une carte de circuit imprimé distincte de celle sur laquelle l'accéléromètre peut être monté.

Avantageusement, la pédale connectée comporte une unité de communication sans-fil apte à recevoir ladite valeur déterminée par ladite unité de calcul et agencée pour transmettre une information relative à cette valeur à un terminal distant. Le cas échéant, ladite unité de communication sans-fil peut être montée sur la deuxième carte de circuit imprimé.

Ladite unité de calcul peut être agencée pour réaliser un calcul périodique de ladite valeur. L'unité de calcul peut être adaptée pour transmettre cette valeur à l'unité de communication sans fil à chaque fois que ladite unité de calcul est connectée à ladite unité de communication sans fil. De préférence, l'unité de communication sans fil peut être apte à échanger des données avec le terminal distant selon un protocole donné, par exemple un protocole WiFi® ou un protocole Bluetooth® ou un protocole ANT+. Le terminal distant peut être un compteur GPS, une montre connectée, un téléphone intelligent, un ordinateur ou une tablette, permettant le suivi des données.

Avantageusement, le gyromètre est apte à emprunter une configuration d'acquisition dans laquelle il est apte à mesurer une rotation de ladite pédale et une configuration éteinte, en ce que chacune des jauges de déformation est apte à emprunter une configuration d'acquisition dans laquelle elle est apte à mesurer une déformation subie par un corps d'épreuve de cette jauge et une configuration éteinte, en ce que l'accéléromètre est apte à mesurer une accélération de ladite pédale, et en ce qu'elle comporte une unité de contrôle agencée pour, lors de la détection d'une accélération par l'accéléromètre, contrôler le gyromètre pour qu'il emprunte la configuration d'acquisition et pour, lors de la détection d'une rotation par le gyromètre, contrôler les jauges de déformation pour qu'elles empruntent simultanément leurs configurations d'acquisition.

L'accéléromètre peut être apte à détecter une accélération. Le gyromètre peut être apte à détecter une rotation de la pédale. L'unité de contrôle peut contrôler le gyromètre pour qu'il emprunte la configuration d'acquisition lorsque l'accéléromètre mesure une accélération supérieure à un seuil donné. L'unité de contrôle peut contrôler les jauges de déformation pour qu'elles empruntent simultanément leur configuration d'acquisition lorsque le gyromètre détecte un nombre de rotations supérieur à un seuil donné.

La configuration d'acquisition des données par les deux jauges de déformation est maintenue tant que le gyromètre détecte une rotation de la pédale et que l'accéléromètre détecte une accélération. A l'inverse, lorsque le gyromètre et l'accéléromètre ne détectent respectivement aucune giration et aucune accélération pendant un temps donné, notamment supérieur à 5 secondes, les deux jauges de déformation adoptent alors la configuration éteinte. Cette caractéristique peut notamment permettre d'optimiser la consommation électrique de la pédale connectée, en permettant à ladite pédale d'adopter un mode moins énergivore en cas d'inactivité du gyromètre, de l'accéléromètre et des jauges de déformation.

Avantageusement, la plateforme est reliée à une manivelle et l'unité de calcul de la pédale est agencée pour déterminer une orientation de l'axe creux de la pédale vis-à-vis de la manivelle.

Le cas échéant, ladite manivelle peut être montée, via une liaison pivot, sur un cadre du vélo.

La détermination de l'orientation de l'axe creux de la pédale vis-à-vis de la manivelle peut permettre une calibration de l'unité de calcul, de manière à pouvoir déterminer ladite force exercée par un cycliste sur la pédale de façon répétable et fiable, quelle que soit la manière dont la pédale ait été fixée sur la manivelle.

Avantageusement, afin de déterminer ladite orientation de l'axe creux, l'unité de calcul est agencée pour déterminer la valeur d'un angle de décalage entre une direction normale à l'une des surfaces de l'axe creux et la verticale. Il est à noter que la valeur de cet angle correspond au décalage entre une force motrice de la pédale et la verticale. Cet angle peut ainsi indiquer la position des jauges de déformation par rapport à la verticale

Dans un exemple de réalisation, ladite unité de communication sans-fil peut être apte à recevoir une instruction de calibration de l'unité de calcul, par exemple émis par le terminal distant, notamment à l'issue d'une action manuelle exercée sur le terminal distant par un utilisateur. A la réception de ladite instruction de calibration, l'unité de communication sans-fil est agencée pour transmettre ladite instruction à l'unité de calcul, laquelle est agencée pour commander lesdites jauges de déformation pour réaliser une mesure initiale d'une force exercée sur la pédale.

Si on le souhaite, l'unité de calcul est alors agencée pour tarer les mesures initiales réalisées par lesdites jauges de déformation, une valeur de 0 Newton étant attribuée auxdites mesures initiales. Le cas échéant, pour chaque mesure ultérieure réalisée par lesdites jauges de déformation, ladite unité de calcul est agencée pour convertir cette mesure en une valeur calibrée représentant une force estimée en Newton en tenant compte des valeurs tarées des mesures initiales.

Avantageusement, l'unité de calcul est agencée pour déterminer la valeur de l'angle au moyen de l'équation suivante :

[Math. 1]
$$\vec{g} = \cos\alpha \times \overrightarrow{acc_y} + \sin\alpha \times \overrightarrow{acc_z,}$$

où g est la valeur de la force de gravité, $\alpha$ est la valeur de l'angle de décalage, $acc_y$ et $acc_z$ sont les mesures initiales réalisées par les jauges de déformation.

Il est à relever que dans le cas où la pédale est libre de toute masse, où l'axe creux est vissé à son maximum avec sa plateforme montée dessus et où la manivelle est orientée verticalement, seule la force de gravité s'exerce sur les jauges de la pédale. Dès lors, dans cette configuration, l'équation ci-dessus est vérifiée quelle que soit l'orientation de l'axe vis-à-vis de la manivelle. La force de gravité g étant connue, il est donc possible de déterminer l'angle de décalage. L'action manuelle de l'utilisateur devra avantageusement être précédée d'une orientation manuelle de la manivelle dans ladite configuration.

Avantageusement, l'unité de calcul est agencée pour déterminer une valeur d'une force motrice de la pédale à partir des mesures acquises par les jauges de déformation et de ladite valeur de l'angle de décalage. De préférence, l'unité de calcul est agencée pour déterminer ladite valeur de la force motrice de la pédale à partir des valeurs calibrées obtenues à partir des mesures acquises par les jauges de déformation et de ladite valeur de l'angle de décalage. Le cas échéant, l'unité de calcul peut être agencée pour déterminer ladite au moins une autre valeur relative au pédalage du cycliste à partir de ladite valeur de la force motrice.

Avantageusement, la pédale connectée comporte au moins une source d'alimentation électrique logée dans l'axe creux et agencée pour alimenter électriquement les jauges de déformation, le gyromètre et l'unité de calcul.

Dans un mode de réalisation de l'invention, la deuxième carte de circuit imprimé peut comporter un convertisseur d'alimentation électrique agencé pour recevoir une puissance électrique issue de ladite source d'alimentation et pour alimenter, à partir de ladite puissance, ladite unité de calcul, et le cas échéant, la centrale inertielle et l'unité de communication sans-fil. De préférence, la première carte de circuit imprimé peut comporter un convertisseur d'alimentation électrique supplémentaire agencé pour recevoir une puissance électrique issue du convertisseur de la deuxième carte de circuit imprimé et pour alimenter, à partir de ladite puissance, les jauges de déformation, et le cas échéant, les amplificateurs opérationnels. La présence d'un convertisseur supplémentaire permet de fournir une alimentation électrique stabilisée aux jauges de déformation de sorte que les mesures réalisées par ces jauges soient stables et comparables.

Si on le souhaite, l'axe creux peut comporter un orifice via lequel s'étend un faisceau de connexion électrique reliant ladite source d'alimentation électrique au convertisseur de la deuxième carte de circuit imprimé. Avantageusement, la pédale connectée comporte un connecteur logé dans l'axe creux et relié à la source d'alimentation électrique, ledit connecteur étant destiné à être couplé à un organe de rechargement de ladite source d'alimentation électrique.

L'axe étant creux, il peut être possible d'ajouter un connecteur de charge afin de connecter la source d'alimentation en vue de son rechargement.

L'invention a également pour objet une paire de pédales, notamment une pédale gauche et une pédale droite, chaque pédale étant une pédale selon l'invention.

Avantageusement, chaque pédale est munie d'une unité de communication sans-fil, l'une des pédale étant une pédale maitre et l'autre pédale étant une pédale esclave. De préférence, l'unité de communication sans-fil de la pédale esclave est agencée pour transmettre à l'unité de communication sans-fil de la pédale maitre ladite valeur relative à la force exercée par un cycliste sur la pédale esclave déterminée par l'unité de calcul de la pédale esclave, et l'unité de calcul de la pédale maitre est agencée pour déterminer une valeur relative à une force globale exercée par le cycliste sur la paire de pédale à partir desdites valeurs relatives à une force exercée par un cycliste sur la pédale maitre et sur la pédale esclave. Le cas échéant, ladite unité de communication sans-fil de la pédale maitre est apte à recevoir ladite valeur relative à une force globale exercée par le cycliste sur la paire de pédale déterminée par ladite unité de calcul de la pédale maitre et agencée pour transmettre cette valeur à un terminal distant.

L'organe de rechargement peut être un chargeur monté sur la deuxième carte de circuit imprimée.

Brève description des figures.

D'autres avantages et caractéristiques de la présente invention sont maintenant décrits à l'aide d'exemples uniquement illustratifs et nullement limitatifs de la portée de l'invention, et à partir des dessins annexés, dessins sur lesquels les différentes figures représentent :

[Fig. 1] représente schématiquement, une vue de dessus d'une pédale connectée selon un mode de réalisation.

[Fig. 2] représente schématiquement une vue de face d'une pédale connectée selon un mode de réalisation.

[Fig. 3] représente schématiquement une vue de profil d'une pédale connectée selon un mode de réalisation.

[Fig. 4] représente schématiquement une vue en perspective d'un axe creux d'une pédale connectée selon un mode de réalisation.

[Fig. 5] représente schématiquement une vue de profil d'un axe creux d'une pédale connectée selon un mode de réalisation.

[Fig. 6] représente schématiquement une vue en coupe d'un axe creux d'une pédale connectée selon un mode de réalisation.

[Fig. 7] représente schématiquement le fonctionnement d'une pédale connectée selon un mode de réalisation.

[Fig. 8] représente schématiquement le fonctionnement de l'unité de calcul d'un couple de pédales connectées selon un mode de réalisation.

Dans la description qui suit, les éléments identiques, par structure ou par fonction, apparaissant sur différentes figures conservent, sauf précision contraire, les mêmes références.

Description des modes de réalisation.

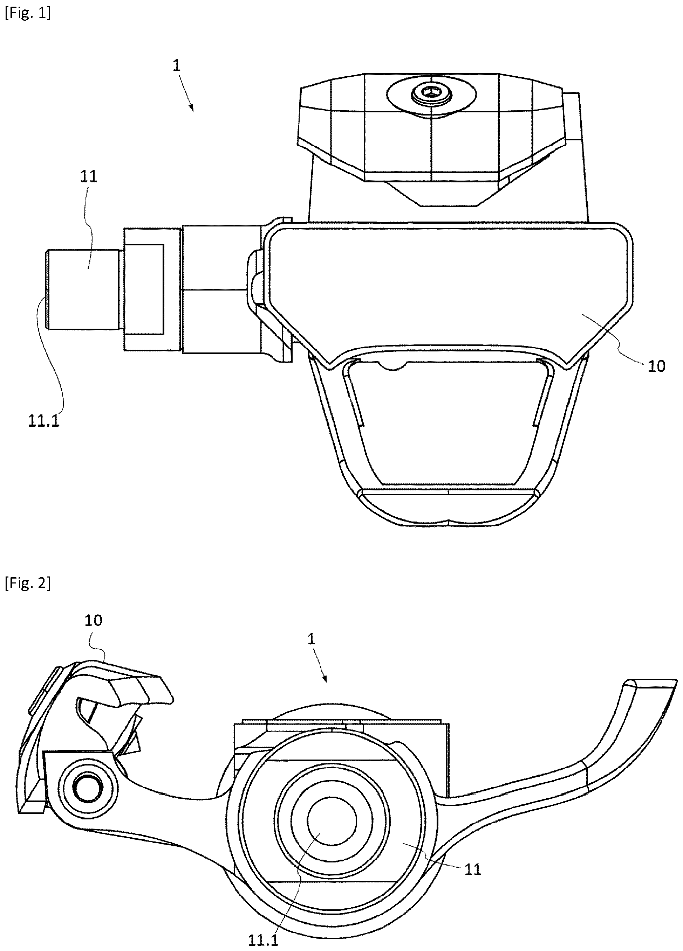

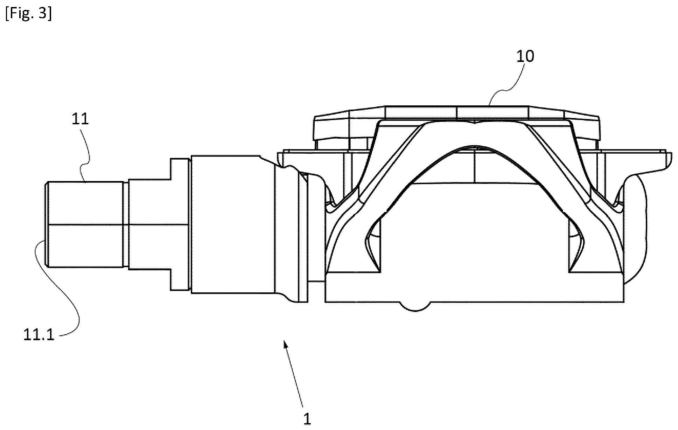

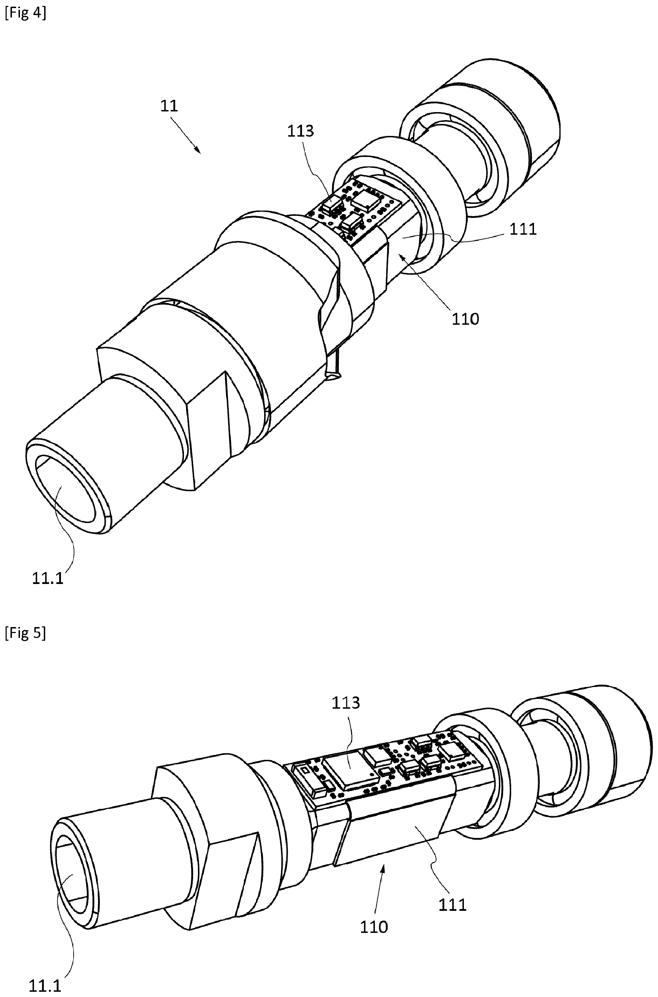

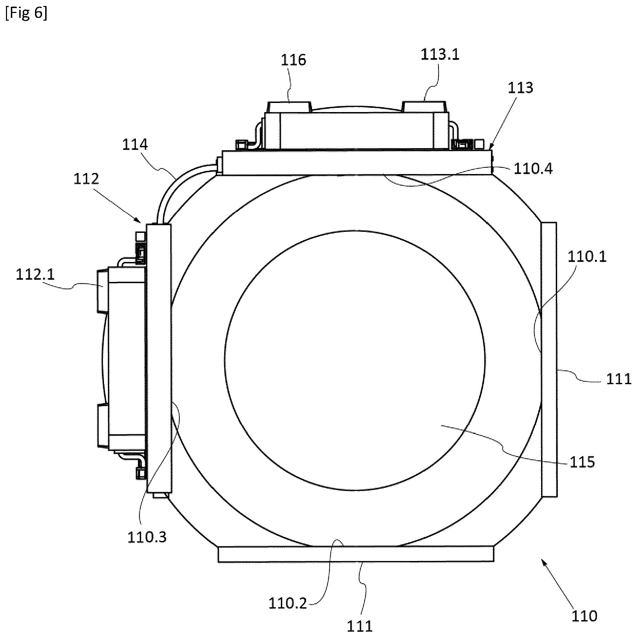

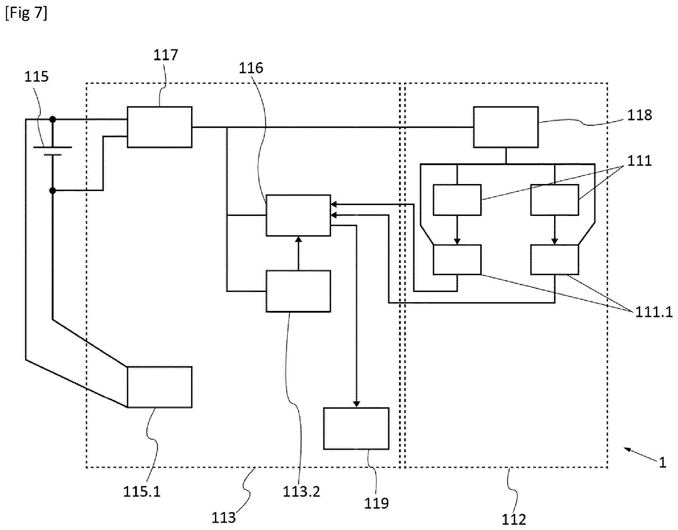

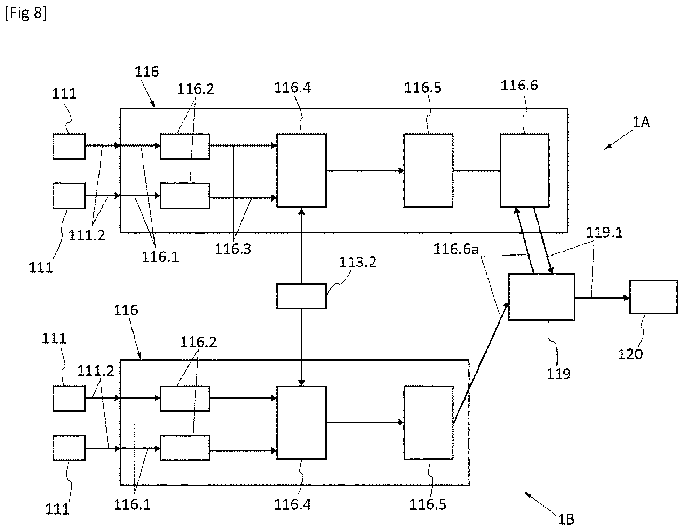

On a représenté en [Fig. 1], [Fig. 2] et [Fig. 3] une pédale 1 connectée de vélo, respectivement en vue de dessus, en vue de face et en vue de profil, décrite en lien avec les [Fig. 7] et [Fig. 8]. La pédale 1 connectée comporte un axe creux décrit en [Fig. 4], [Fig. 5] et [Fig. 6].

La pédale connectée 1 est adaptée pour être montée sur un vélo de manière interchangeable. La pédale 1 connectée comporte une plateforme 10 et l'axe creux 11.

La plateforme 10 forme une pédale plate sur laquelle le pied de l'utilisateur vient reposer. La plateforme 10 est formée de sorte à pouvoir recevoir une chaussure d'un utilisateur et de manière à ce que celle-ci puisse coopérer avec ladite plateforme 10 pour maintenir le pied de l'utilisateur sur ladite plateforme 10.

La plateforme 10 est montée sur l'axe creux 11 de manière amovible. La plateforme 10 et l'axe creux 11 des pédales 1 droites et gauches sont sensiblement identiques. L'axe creux 11 est monté sur la manivelle gauche ou la manivelle droite par un pas de vis 11.1.

La plateforme 10 est montée mobile en rotation sur l'axe creux 11. L'axe creux 11 est interchangeable entre un vélo de type route et un vélo de type tout terrain.

Comme décrit en [Fig. 6] l'axe creux 11 comporte une portion support 11 de section carrée ou rectangulaire. La portion support 110 comporte une section dans laquelle deux faces 110.1, 110.2, 110.3, 110.4 voisines deux à deux forment un angle de 90° l'une par rapport à l'autre. La portion support 110 présente une première face 110.1 et une deuxième face 110.2 perpendiculaires entre elles. La portion support 110 présente une troisième face 110.3 et une quatrième face 110.4, perpendiculaires entre elles et distinctes de la première 110.1 et de la deuxième face 110.2. La troisième face 110.3 est opposée et parallèle à la première face 110.1 et la quatrième face 110.4 est opposée et parallèle à la deuxième face 110.2.

Comme décrit en [Fig. 4], [Fig. 5] et [Fig. 6], la pédale 1 connectée comporte quatre jauges de déformation 111 montées sur la portion support 110 de l'axe creux 11. Les quatre jauges de déformation 111 sont montées deux à deux sur des faces voisines de la portion support 110, par exemple sur la première face 110.1 et sur la deuxième face 110.2. Ainsi deux jauges de déformation 111 sont montées à 90° l'une de l'autre sur la première face 110.1 et deux autres jauges de déformation 111 sont montées à 90° l'une de l'autre sur la deuxième face 110.2. Chacune des jauges de déformation 111 est apte à emprunter une configuration d'acquisition dans laquelle elle est apte à mesurer une déformation subie par un corps d'épreuve de cette jauge 111 et une configuration éteinte.

Comme décrit en [Fig. 4], [Fig. 5] et [Fig. 6], la pédale 1 connectée comporte une première carte de circuit imprimé 112 et une deuxième carte de circuit imprimé 113, montées sur la portion support 110 de l'axe creux 11. La première carte de circuit imprimé 112 et la deuxième carte de circuit imprimé 113 sont montées sur des faces 110.3, 110.4 distinctes des faces 110.1, 110.2 sur lesquelles sont montées les jauges de déformation 111. La première carte de circuit imprimé 112 est montée sur la troisième face 110.3 de la portion support 110 de l'axe creux 11. La deuxième carte de circuit imprimé 113 est montée sur la quatrième face 110.4 de la portion support 110 de l'axe creux 11. Ainsi la première carte de circuit imprimé 112 et la deuxième carte de circuit imprimé 113 sont montées à 90° l'une de l'autre, sur deux faces 110.4, 110.3 distinctes. La première carte de circuit imprimé 112 et la deuxième carte de circuit imprimé 113 sont reliées entre elles par un connecteur flexible 114 de manière à former une pièce unique dont une partie est montée sur la quatrième face 110.4 de la portion support 110 de l'axe creux 11 et une autre partie est montée sur la troisième face 110.3 de la portion support 110 de l'axe creux 11.

La pédale 1 connectée comporte une source d'alimentation électrique 115 logée dans l'axe creux 11 comme décrit en [Fig. 6]. La pédale 1 connectée comporte également un connecteur (non représenté) logé dans l'axe creux 11 et relié à la source d'alimentation électrique 115, le connecteur étant destiné à être couplé à un organe de rechargement 115.1 de ladite source d'alimentation électrique 115.

La pédale 1 connectée comporte un gyromètre 112.1 monté sur la première carte de circuit imprimé 112 sur la troisième face 110.3 de la portion support 110 de l'axe creux 11, et un accéléromètre 113.1 monté sur la deuxième carte de circuit imprimé 113 sur la quatrième face 110.4 de la portion support 110 de l'axe creux 11. Le gyromètre 112.1 détecte une giration de la pédale 1. Le gyromètre 112.1 est apte à emprunter une configuration d'acquisition dans laquelle il est apte à mesurer une rotation de ladite pédale 1 et une configuration éteinte. L'accéléromètre 113.1 détecte une accélération de ladite pédale 1. Le gyromètre 112.1 et l'accéléromètre 113.1 forment une centrale inertielle 113.2.

La pédale 1 connectée comporte une unité de contrôle agencée pour contrôler le gyromètre pour qu'il emprunte la configuration d'acquisition. L'unité de contrôle, contrôle le gyromètre 112.1 pour qu'il emprunte la configuration d'acquisition lorsque l'accéléromètre 113.1 mesure une accélération supérieure à un seuil donné. L'unité de contrôle est également agencée pour contrôler les jauges de déformation 111 pour qu'elles empruntent simultanément leurs configurations d'acquisition. L'unité de contrôle, contrôle les jauges de déformation 111 lors de la détection par le gyromètre 112.1 d'un nombre de rotations supérieur à un seuil donné.

La configuration d'acquisition des données par les deux jauges de déformation 111 est maintenue tant que le gyromètre 112.1 détecte une rotation de la pédale 1 et que l'accéléromètre 113.1 détecte une accélération. A l'inverse, lorsque le gyromètre 112.1 et l'accéléromètre 113.1 ne détectent respectivement aucune giration et aucune accélération pendant un temps donné, par exemple pendant plus de 5 secondes, les deux jauges de déformation 111 adoptent alors la configuration éteinte. Cette caractéristique permet d'optimiser la consommation électrique de la pédale 1 connectée, en permettant à ladite pédale 1 d'adopter un mode moins énergivore en cas d'inactivité du gyromètre 112.1, de l'accéléromètre 113.1 et des jauges de déformation 111.

Le gyromètre 112.1 et l'accéléromètre 113.1 sont adaptés pour coopérer ensemble afin de déterminer l'orientation de l'axe creux 11 de la pédale 1 connectée vis-à-vis de la manivelle et ainsi permettre une calibration de la première unité de calcul et de la deuxième unité de calcul. La détermination de l'orientation de l'axe creux 11 de la pédale 1 vis-à-vis de la manivelle entraine une calibration de l'unité de calcul 116, de manière à pouvoir déterminer ladite force exercée par un cycliste sur la pédale.

L'unité de calcul 116 est agencée pour déterminer la valeur d'un angle de décalage entre une direction normale à l'une des surfaces de l'axe creux 11 et la verticale.

L'unité de communication 119 sans-fil est apte à recevoir une instruction de calibration de l'unité de calcul 116, émis par un terminal distant 120 à l'issue d'une action manuelle exercée sur le terminal distant 120 par un utilisateur, ledit terminal distant 120 étant un compteur GPS. A la réception de ladite instruction de calibration, l'unité de communication 119 sans-fil est agencée pour transmettre ladite instruction à l'unité de calcul 116, laquelle est agencée pour commander lesdites jauges de déformation 111 pour réaliser une mesure initiale d'une force exercée sur la pédale. L'unité de calcul 116 est alors agencée pour tarer les mesures initiales réalisées par lesdites jauges de déformation 111, une valeur de 0 Newton étant attribuée auxdites mesures initiales. Le cas échéant, pour chaque mesure ultérieure réalisée par lesdites jauges de déformation 111, ladite unité de calcul 116 est agencée pour convertir cette mesure en une valeur calibrée représentant une force estimée en Newton en tenant compte des valeurs tarées des mesures initiales.

Comme décrit en [Fig. 7] et [Fig. 8], la première carte de circuit imprimé 112 est équipée de deux amplificateurs opérationnels 111.1, aptes à amplifier des signaux émis par les quatre jauges de déformation 111. La deuxième carte de circuit imprimé 113 est équipée de ladite unité de calcul 116, laquelle est agencée pour convertir lesdits signaux amplifiés issus de la première carte de circuit imprimé 112, ces signaux étant des signaux analogiques 111.2, en des signaux numériques 116.1 et pour déterminer ladite valeur relative à une force 116.5 exercée par un cycliste sur la pédale 1 à partir desdits signaux numériques 116.1. Lesdits signaux numériques 116.1 étant calibrés, dans une étape116.2 par l'unité de calcul 116, de manière à leur attribuer une valeur de force 116.3 en Newton.

L'unité de calcul 116 est agencée pour déterminer, dans une étape 116.4, à partir desdits signaux numériques 116.1, une ou plusieurs valeurs relatives 116.5, notamment, une valeur relative à une force exercée par un cycliste sur la pédale, une valeur relative au pédalage du cycliste, une valeur relative à une cadence de pédalage du cycliste, une valeur relative à l'efficacité du cycliste et/ou une valeur relative à la fluidité du coup de pédale.

La deuxième carte de circuit imprimé 113 comporte un convertisseur d'alimentation électrique 117 agencé pour recevoir une puissance électrique issue de ladite source d'alimentation 115 et pour alimenter, à partir de ladite puissance, ladite unité de calcul 116, la centrale inertielle 113.2 et l'unité de communication sans-fil 119. De préférence, la première carte de circuit imprimé 112 comporte un convertisseur d'alimentation électrique supplémentaire 118 agencé pour recevoir une puissance électrique issue du convertisseur 117 de la deuxième carte de circuit imprimé 113 et pour alimenter, à partir de ladite puissance, les jauges de déformation 111 et les amplificateurs opérationnels 111.1.

L'axe creux 11 comporte un orifice (non représenté) via lequel s'étend un faisceau de connexion électrique reliant ladite source d'alimentation électrique 115 au convertisseur 117 de la deuxième carte de circuit imprimé 113.

La [Fig. 8] décrit le procédé de fonctionnement d'une paire de pédales 1 dans laquelle l'une des pédales 1 est une pédale maitre 1A et l'autre pédale 1 est une pédale esclave 1B.

L'unité de communication sans-fil 119 de la pédale esclave 1B est agencée pour transmettre dans une étape 116.6a à la pédale maitre 1A, par l'unité de communication sans-fil 119, ladite valeur relative 116.5 à la force exercée par un cycliste sur la pédale esclave 1B déterminée par l'unité de calcul 116 de la pédale esclave 1B. L'unité de calcul 116 de la pédale maitre 1A est agencée pour déterminer une valeur relative à une force globale 116.6 exercée par le cycliste sur la paire de pédales 1A, 1B à partir desdites valeurs relatives 116.5 à une force exercé par un cycliste sur la pédale maitre 1A et sur la pédale esclave 1B. Ladite unité de communication 119 sans-fil de la pédale maitre 1Aest apte à recevoir dans une étape 119.1, ladite valeur relative à une force globale 116.6 exercée par le cycliste sur la paire de pédale 1A, 1B déterminée par ladite unité de calcul 116 de la pédale maitre 1A et est agencé pour transmettre dans une étape 119.1 cette valeur au terminal distant 120. La description qui précède explique clairement comment l'invention permet d'atteindre les objectifs qu'elle s'est fixée, à savoir proposer une pédale connectée comportant un axe standard et intervertible d'un type de vélo à un autre et dont la conception est simplifiée et peu énergivore, en proposant une pédale connectée de vélo comportant un corps comportant une plateforme formant une pédale, un axe creux sur lequel la plateforme est montée mobile en rotation, l'axe comportant une portion support de section carrée ou rectangulaire, la pédale étant remarquable en ce qu'elle comporte deux jauges de déformation montées sur l'axe creux, les jauges étant montées sur des faces voisines de ladite portion support, lesdites faces voisines étant montées à 90° l'une de l'autre, lesdites faces voisines étant appelées première face et deuxième faces, et au moins un gyromètre monté sur une troisième face de ladite portion support de l'axe creux, cette troisième face étant distincte des première et deuxième faces supportant les jauges de déformation ; et la pédale étant remarquable en ce qu'elle comporte au moins une unité de calcul agencée pour déterminer une valeur relative à une force exercée par un cycliste sur la pédale à partir des mesures acquises par les jauges de déformation et le gyromètre. En tout état de cause, la portée de l'invention est définie par les revendications.

## Revendications

1. Pédale (1) connectée de vélo comportant un corps comportant :

   a. Une plateforme (10) formant une pédale (1),
   b. Un axe creux (11) sur lequel la plateforme (10) est montée mobile en rotation, l'axe (11) comportant une portion support (110) de section carrée ou rectangulaire ;
   **caractérisée en ce qu'**elle comporte deux jauges de déformation (111) montées sur l'axe creux (11), les jauges (111) étant montées sur des faces voisines de ladite portion support (110), lesdites faces voisines étant montées à 90° l'une de l'autre, lesdites faces voisines étant appelées première face (110.1) et deuxième face (110.2), et au moins un gyromètre (112.1) monté sur une troisième face (110.3) de ladite portion support (110) de l'axe creux (11), cette troisième face (110.3) étant distincte des première (110.1) et deuxième face (110.2) supportant les jauges de déformation (111) ; et **en ce qu'**elle comporte au moins une unité de calcul (116) agencée pour déterminer une valeur relative (116.5) à une force exercée par un cycliste sur la pédale (1) à partir des mesures acquises par les jauges de déformation (111) et le gyromètre (112.1).

2. Pédale (1) connectée selon la revendication 1, **caractérisée en ce qu'**elle comporte quatre jauges de déformation (111) montées sur l'axe creux (11), deux desdites jauges de déformation (111) étant montées sur la première face (110.1) en faisant un angle entre elles et deux autres desdites jauges de déformation (111) étant montées sur la deuxième face (110.2) en faisant un angle entre elles.

3. Pédale (1) connectée selon l'une des revendications précédentes, **caractérisée en ce qu'**elle comporte une première carte de circuit imprimé (112) et une deuxième carte de circuit imprimé (113), montées sur l'axe creux (11), la première carte de circuit imprimé (112) étant montée sur la troisième face (110.3) de la portion support (110) et la deuxième carte de circuit imprimé (113) étant montée sur une quatrième face (110.4) de la portion support (110), la troisième (110.3) et la quatrième face (110.4) étant voisines.

4. Pédale (1) connectée selon la revendication précédente, **caractérisée en ce que** la première carte de circuit imprimé (112) est équipée d'un ou plusieurs composants électroniques (111.1) aptes à amplifier des signaux émis par lesdites jauges de déformation (111) et la deuxième carte de circuit imprimé (113) est équipée d'au moins ladite unité de calcul (116), laquelle est agencée pour convertir lesdits signaux amplifiés issus de la première carte de circuit imprimé (112), ces signaux étant des signaux analogiques (111.2) , en des signaux numériques (116.1) et pour déterminer ladite valeur relative (116.5) à une force exercée par un cycliste sur la pédale (1) à partir desdits signaux numériques (116.1).

5. Pédale (1) connectée selon l'une des revendications 3 ou 4, **caractérisée en ce que** le gyromètre (112.1) est monté sur la première carte de circuit imprimé (112) et **en ce qu'**elle comporte un accéléromètre (113.1) monté sur la deuxième carte de circuit imprimé (113).

6. Pédale (1) connectée selon l'une des revendications précédentes, **caractérisée en ce qu'**elle comporte une unité de communication (119) sans-fil apte à recevoir ladite valeur déterminée par ladite unité de calcul (116) et agencée pour transmettre une information relative à cette valeur à un terminal distant (120).

7. Pédale (1) connectée selon la revendication 5 ou la revendication 6 prise en combinaison avec la revendication 5, **caractérisée en ce que** le gyromètre (112.1) est apte à emprunter une configuration d'acquisition dans laquelle il est apte à mesurer une rotation de ladite pédale (1) et une configuration éteinte, **en ce que** chacune des jauges de déformation (111) est apte à emprunter une configuration d'acquisition dans laquelle elle est apte à mesurer une déformation subie par un corps d'épreuve de cette jauge (111) et une configuration éteinte, **en ce que** l'accéléromètre (113.1) est apte à mesurer une accélération de ladite pédale (1), et **en ce qu'**elle comporte une unité de contrôle agencée pour, lors de la détection d'une accélération par l'accéléromètre (113.1), contrôler le gyromètre (112.1) pour qu'il emprunte la configuration d'acquisition et pour, lors de la détection d'une rotation par le gyromètre (112.1), contrôler les jauges de déformation (111) pour qu'elles empruntent simultanément leurs configurations d'acquisition.

8. Pédale (1) connectée selon l'une des revendications précédentes, **caractérisée en ce que** la plateforme (10) est reliée à une manivelle et **en ce que** l'unité de calcul (116) de la pédale (1) est agencée pour déterminer une orientation de l'axe creux (11) de la pédale (1) vis-à-vis de la manivelle.

9. Pédale (1) connectée selon l'une des revendications précédentes, **caractérisée en ce qu'**elle comporte au moins une source d'alimentation électrique (115) logée dans l'axe creux (11) et agencée pour alimenter électriquement les jauges de déformation (111), le gyromètre (112.1) et l'unité de calcul.

10. Pédale (1) connectée selon la revendication précédente, **caractérisée en ce qu'**elle comporte un connecteur (114) logé dans l'axe creux (11) et relié à la source d'alimentation électrique (115), ledit connecteur (114) étant destiné à être couplé à un organe de rechargement (115.1) de ladite source d'alimentation électrique (115).

11. Paire de pédales (1A, 1B), **caractérisée en ce que** chaque pédale (1A, 1B) est une pédale (1) selon l'une des revendications précédentes.

## Patentansprüche

1. Vernetztes Fahrradpedal (1), umfassend einen Körper, umfassend:

   a. eine Plattform (10), die ein Pedal (1) bildet,
   b. eine hohle Achse (11), auf der die Plattform (10) drehbeweglich montiert ist, wobei die Achse (11) einen Trägerabschnitt (110) mit einem quadratischen oder rechteckigen Abschnitt umfasst;

   **dadurch gekennzeichnet, dass** es zwei Verformungsmessstreifen (111) umfasst, die auf der hohlen Achse (11) montiert sind, wobei die Messstreifen (111) auf benachbarten Flächen des Trägerabschnitts (110) montiert sind, wobei die benachbarten Flächen um 90° zueinander montiert sind, wobei die benachbarten Flächen erste Fläche (110.1) und zweite Fläche (110.2) genannt werden, und mindestens ein Gyrometer (112.1), das auf einer dritten Fläche (110.3) des Trägerabschnitts (110) der hohlen Achse (11) montiert ist, wobei diese dritte Fläche (110.3) sich von der ersten (110.1) und zweiten Fläche (110.2), welche die Verformungsmessstreifen (111) tragen, unterscheidet; **und dadurch, dass** es mindestens eine Recheneinheit (116) umfasst, die angeordnet ist, um einen Wert (116.5) in Bezug auf einer von einem Radfahrer auf das Pedal (1) ausgeübten Kraft aus den von den Verformungsmessstreifen (111) und dem Gyrometer (112.1) erfassten Messungen zu bestimmen.

2. Vernetztes Pedal (1) nach Anspruch 1, **dadurch gekennzeichnet, dass** es vier Verformungsmessstreifen (111) umfasst, die auf der hohlen Achse (11) montiert sind, wobei zwei der Verformungsmessstreifen (111) auf der ersten Fläche (110.1) montiert sind und einen Winkel zwischen ihnen bilden, und zwei andere der Verformungsmessstreifen (111) auf der zweiten Fläche (110.2) montiert sind und einen Winkel zwischen ihnen bilden.

3. Vernetztes Pedal (1) nach einem der vorstehenden Ansprüche, **dadurch gekennzeichnet, dass** es eine erste Leiterplatte (112) und eine zweite Leiterplatte (113) umfasst, die auf der hohlen Achse (11) montiert sind, wobei die erste Leiterplatte (112) auf der dritten Fläche (110.3) des Trägerabschnitts (110) montiert ist, und die zweite Leiterplatte (113) auf einer vierten Fläche (110.4) des Trägerabschnitts (110) montiert ist, wobei die dritte (110.3) und die vierte Fläche (110.4) benachbart sind.

4. Vernetztes Pedal (1) nach dem vorstehenden Anspruch, **dadurch gekennzeichnet, dass** die erste Leiterplatte (112) mit einem oder mehreren elektronischen Komponenten (111.1) ausgestattet ist, die dazu geeignet sind, von den Verformungsmessstreifen (111) ausgesendete Signale zu verstärken, und die zweite Leiterplatte (113) mit mindestens der Recheneinheit (116) ausgestattet ist, die angeordnet ist, um die verstärkten Signale von der ersten Leiterplatte (112), wobei diese Signale analoge Signale (111.2) sind, in digitale Signale (116.1) umzuwandeln und den relativen Wert (116.5) zu einer von einem Radfahrer auf das Pedal (1) ausgeübten Kraft aus den digitalen Signalen (116.1) zu bestimmen.

5. Vernetztes Pedal (1) nach einem der Ansprüche 3 oder 4, **dadurch gekennzeichnet, dass** das Gyrometer (112.1) auf der ersten Leiterplatte (112) montiert ist, **und dadurch,** dass es einen Beschleunigungsmesser (113.1) umfasst, der auf der zweiten Leiterplatte (113) montiert ist.

6. Vernetztes Pedal (1) nach einem der vorstehenden Ansprüche, **dadurch gekennzeichnet, dass** es eine drahtlose Kommunikationseinheit (119) umfasst, die dazu geeignet ist, den von der Berechnungseinheit (116) bestimmten Wert zu empfangen und angeordnet ist, um eine Information in Bezug auf diesen Wert an ein entferntes Endgerät (120) zu übertragen.

7. Vernetztes Pedal (1) nach Anspruch 5 oder Anspruch 6 in Kombination mit Anspruch 5, **dadurch gekennzeichnet, dass** das Gyrometer (112.1) dazu geeignet ist, eine Erfassungskonfiguration, in der es geeignet ist, eine Drehung des Pedals (1) zu messen, und eine ausgeschaltete Konfiguration anzunehmen, **dadurch,** dass jeder der Verformungsmessstreifen (111) dazu geeignet ist, eine Erfassungskonfiguration, in der er dazu geeignet ist, eine Verformung zu messen, die einem Prüfkörper dieses Messstreifens (111) unterliegt, und eine ausgeschaltete Konfiguration anzunehmen, **dadurch,** dass der Beschleunigungsmesser (113.1) dazu geeignet ist, eine Beschleunigung des Pedals (1) zu messen, **und dadurch,** dass es eine Steuereinheit umfasst, die angeordnet ist, um während der Detektion einer Beschleunigung durch den Beschleunigungsmesser (113.1) das Gyrometer (112.1) zu steuern, damit es die Erfassungskonfiguration annimmt, und um während der Detektion einer Drehung durch das Gyrometer (112.1) die Verformungsmessstreifen (111) zu steuern, damit sie gleichzeitig ihre Erfassungskonfigurationen annehmen.

8. Vernetztes Pedal (1) nach einem der vorstehenden Ansprüche, **dadurch gekennzeichnet, dass** die Plattform (10) mit einer Kurbel verbunden ist, **und dadurch, dass** die Recheneinheit (116) des Pedals (1) angeordnet ist, um eine Ausrichtung der hohlen Achse (11) des Pedals (1) in Bezug auf die Kurbel zu bestimmen.

9. Vernetztes Pedal (1) nach einem der vorstehenden Ansprüche,
**dadurch gekennzeichnet, dass** es mindestens eine elektrische Versorgungsquelle (115) umfasst, die in der hohlen Achse (11) untergebracht und angeordnet ist, um die Verformungsmessstreifen (111), das Gyrometer (112.1) und die Recheneinheit elektrisch zu versorgen.

10. Vernetztes Pedal (1) nach dem vorstehenden Anspruch,
**dadurch gekennzeichnet, dass** es einen Verbinder (114) umfasst, der in der hohlen Achse (11) untergebracht und mit der elektrischen Versorgungsquelle (115) verbunden ist, wobei der Verbinder (114) dazu bestimmt ist, an ein Aufladeelement (115.1) der elektrischen Versorgungsquelle (115) gekoppelt zu werden.

11. Pedalpaar (1A,1B), **dadurch gekennzeichnet, dass** jedes Pedal (1A, 1B) ein Pedal (1) nach einem der vorstehenden Ansprüche ist.

**Claims**

1. Connected pedal (1) for bicycles, which pedal comprises a body having:

    a. a platform (10) forming a pedal (1),
    b. a hollow shaft (11) on which the platform (10) is mounted so as to be rotatably movable, the shaft (11) comprising a support portion (110) having a square or rectangular cross section;

    **characterized in that** the pedal comprises two strain gauges (111) mounted on the hollow shaft (11), the gauges (111) being mounted on adjacent faces of said support portion (110), said adjacent faces being mounted at 90° to one another, said adjacent faces being referred to as first face (110.1) and second face (110.2), and at least one gyrometer (112.1) mounted on a third face (110.3) of said support portion (110) of the hollow shaft (11), this third face (110.3) being separate from the first face (110.1) and the second face (110.2) supporting the strain gauges (111); **and in that** the pedal comprises at least one computing unit (116) arranged to determine a value (116.5) relative to a force exerted by a cyclist on the pedal (1) on the basis of measurements acquired by the strain gauges (111) and the gyrometer (112.1).

2. Connected pedal (1) according to claim 1, **charac-** terized in that the pedal comprises four strain gauges (111) mounted on the hollow shaft (11), two of said strain gauges (111) being mounted on the first face (110.1) and forming an angle therebetween and another two of said strain gauges (111) being mounted on the second face (110.2) and forming an angle therebetween.

3. Connected pedal (1) according to any of the preceding claims, **characterized in that** the pedal comprises a first printed circuit board (112) and a second printed circuit board (113) which are mounted on the hollow shaft (11), the first printed circuit board (112) being mounted on the third face (110.3) of the support portion (110) and the second printed circuit board (113) being mounted on a fourth face (110.4) of the support portion (110), the third face (110.3) and the fourth face (110.4) being adjacent.

4. Connected pedal (1) according to the preceding claim,
**characterized in that** the first printed circuit board (112) is provided with one or more electronic components (111.1) capable of amplifying signals emitted by said strain gauges (111) and the second printed circuit board (113) is provided with at least said computing unit (116) which is arranged to convert said amplified signals from the first printed circuit board (112), these signals being analog signals (111.2), into digital signals (116.1) and to determine said value (116.5) relative to a force exerted by a cyclist on the pedal (1) from said digital signals (116.1).

5. Connected pedal (1) according to either of claims 3 or 4,
**characterized in that** the gyrometer (112.1) is mounted on the first printed circuit board (112) **and in that** the pedal comprises an accelerometer (113.1) mounted on the second printed circuit board (113).

6. Connected pedal (1) according to any of the preceding claims,
**characterized in that** the pedal comprises a wireless communication unit (119) which is capable of receiving said value determined by said computing unit (116) and which is arranged to transmit information relating to this value to a remote terminal (120).

7. Connected pedal (1) according to claim 5 or claim 6 taken in conjunction with claim 5, **characterized in that** the gyrometer (112.1) is capable of adopting an acquisition configuration in which it is capable of measuring a rotation of said pedal (1), and an off configuration, **in that** each of the strain gauges (111) is capable of adopting an acquisition configuration in which it is capable of measuring a strain undergone by a proof body of this gauge (111), and an off configuration, **in that** the accelerometer (113.1) is capable of measuring an acceleration of said pedal (1), **and in that** the pedal comprises a control unit arranged to control the gyrometer (112.1) when an acceleration is detected by the accelerometer (113.1), so that the gyrometer adopts the acquisition configuration, and arranged to control the strain gauges (111) so that they simultaneously adopt the acquisition configurations thereof when the gyrometer (112.1) detects a rotation.

8.  Connected pedal (1) according to any of the preceding claims,
    **characterized in that** the platform (10) is connected to a crank **and in that** the computing unit (116) of the pedal (1) is arranged to determine an orientation of the hollow shaft (11) of the pedal (1) with respect to the crank.

9.  Connected pedal (1) according to any of the preceding claims,
    **characterized in that** the pedal comprises at least one electrical power supply source (115) received in the hollow shaft (11) and arranged to supply electrical power to the strain gauges (111), the gyrometer (112.1) and the computing unit.

10. Connected pedal (1) connected according to the preceding claim,
    **characterized in that** the pedal comprises a connector (114) received in the hollow shaft (11) and connected to the electrical power supply source (115), said connector (114) being intended to be coupled to a recharging member (115.1) of said electrical power supply source (115).

11. Pair of pedals (1A, 1B), **characterized in that** each pedal (1A, 1B) is a pedal (1) according to any of the preceding claims.

[Fig. 1]

[Fig. 2]

[Fig. 3]

[Fig 4]

11

113

111

110

11.1

[Fig 5]

113

111

110

11.1

[Fig 6]

[Fig 7]

[Fig 8]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3078158 A1